# EUROPEAN PATENT SPECIFICATION

(11) **EP 1 700 619 B1**
(45) Date of publication and mention of the grant of the patent: **16.06.2010**
(21) Application number: 06004717.2
(22) Date of filing: 08.03.2006
(51) Int. Cl.: A61Q 19/10, A61Q 5/02, C11D 17/00

(54) **Detergent composition**
Waschmittel
Composition détersive

(30) Priority: 08.03.2005 JP 2005063593
(43) Date of publication of application: 13.09.2006
(73) Proprietor: KAO CORPORATION, Tokyo 103-8210 (JP)
(72) Inventor: Fujii, Ryosuke, Wakayama-shi Wakayama 640-8580 (JP); Tamura, Yoshinori, Wakayama-shi Wakayama 640-8580 (JP)
(74) Representative: HOFFMANN EITLE

(56) References cited:
- EP-A- 1 221 474
- US-A- 4 537 604
- US-B1- 6 294 509

## Description

### [Technical Field]

The present invention relates to a detergent composition which contains disintegrating particles causing less skin damage or itching because they break up during cleansing or rinsing, is excellent in detergency, and can be washed away sufficiently owing to disintegration of particles by rinse water or tears.

### [Background Art]

Rinse-off skin detergents (including face wash, body shampoo, massage cream, solid soap) containing particles (scrub agent) therein is characterized in that they can readily exfoliate the dead skin cells (grime) or remove dirt from pores by physical cleansing, unlike ordinary detergent compositions.

A mild detergent with high detergency which contains scrubbing particles has been put on the market. The scrubbing particles therein are designed to break up in consideration of not only problems such as irritation to the skin and roughening of the skin but also safety at the time when the scrubbing particles get in eyes.

JP-A-4-198116 and JP-A-5-221826 disclose a detergent which contains particles. These particles dissolve and collapse by the friction of hands or by the action of water during cleansing because they are formed by binding fine powders with a water-soluble binder.

On the other hand, JP-A-2000-63899 discloses a detergent containing particles which easily break up during rinsing or when they get in eyes, because these particles are formed by binding primary particles including water-insoluble particles with a water-soluble binder and their disintegration is controlled by the concentration of a salt.

US 6,294,509 B1 relates to disintegrating particles and a detergent composition containing the same. The disintegrating particles comprise primary particles and a water-soluble binder, wherein said primary particles are agglomerated by said water-soluble binder, the average particle diameter of the primary particles is at most 70 µm, the average particle size of said disintegrating particles ranges from 150 to 360 µm, the rate of disintegration of the disintegrating particles in 10% saline solution is 6.8% to 22.8%, and at least 60 vol.% of the particles are disintegrated in an aqueous solution containing sodium chloride at a concentration lower than 1.5 wit.%. Furthermore, the detergent composition of US 6,294,509 B1 comprises the above-mentioned disintegrating particles, a water-soluble salt, a surfactant and water, wherein the water-soluble salt is an inorganic salt, and the inorganic salt is present at a concentration of from 1.5 wt.% to less than the saturated solubility of the inorganic salt.

US 4,537,604 relates to scouring cleaning compositions comprising from 5 to 99 % by weight of an abrasive agent consisting of agglomerates of finely divided abrasive material having no particles of initial size above 20 µm, at least 80% by weight thereof being of initial size below 10 µm, and an organic binder for agglomerating such material, from 1 to 20 % by weight of the binder being an oil-soluble anionic or cationic polar compound; the flexural strength of the agglomerates being above 1.5 kilograms.

Any known detergents containing disintegrating particles however leave a problem such as foreign-body sensation during massage using the detergents.

### [Summary of the Invention]

The present inventors have investigated the problems and defects of the above-described conventional art. As a result, it has been found that a detergent composition having high detergency and excellent feeling upon use without giving any foreign-body sensation during massage using the compositions can be obtained by incorporating disintegrating particles having an average particle size of not less than 30 µm but less than 100 µm in a detergent, leading to the completion of the present invention.

According to the present invention, there is thus provided a detergent composition comprising the following components (A), (B), (C) and (D):
(A) disintegrating particles for detergent obtained by binding and agglomerating primary particles at least a part of which is water-insoluble with a water-soluble binder and having an average particle size of not less than 30 µm but less than 100 µm,
(B) a water-soluble salt, as defined in claim 1, wherein the water-soluble salt is contained in an amount of not less than 1.5 wt.%,
(C) a surfactant (except for a glyceryl ether), and
(D) water.

### [Detailed Description of the Invention]

The detergent composition according to the present invention is particularly excellent in physical (mechanical) detergency, has excellent time-dependent stability, gives good feeling upon use and, in particular, does not give foreign-body sensation during massage. Moreover, since the disintegrating particles disintegrate during cleansing and rinsing, the detergent composition causes less skin damage or itching and since the disintegrating particles easily disintegrate by the action of rinse water or tears, the composition can be washed away sufficiently.

The disintegrating particles as Component (A) of the present invention are particles formed by agglomeration of primary particles at least a part of which is water-insoluble. The agglomerated particles disintegrate owing to lowering of the concentration of the water-soluble salt in an aqueous solution containing the water-soluble salt. In other words, the particles exhibit a higher disintegration ratio as the concentration of the water-soluble salt lowers.

The primary particles constituting the disintegrating particles of the present invention may be any particles as long as at least a part of which is water-insoluble. For example, water-insoluble primary particles or a combination of water-insoluble primary particles and water-soluble primary particles is preferred. These primary particles may be either organic particles or inorganic particles. The term "water-insoluble" as used herein means that the solubility of the particles at 25°C is less than 0.5 g in 100 g of water, while the term "water-soluble" means that the solubility of the particles at 25°C is not less than 0.5 g in 100 g of water. The solubility is determined from a solid content in a filtrate obtained by filtering an aqueous solution of the particles through a filter paper (No. 2). As the water-soluble primary particles, those having a solubility of not less than 0.9 in 100 g of water are preferred.

Preferred examples of the water-insoluble organic primary particles include synthetic polymers, for example, polyethylenes, polypropylenes, polyamides, polyethylene terephthalates, polystyrenes, polyurethanes and/or crosslinked products thereof, sodium poly(meth)acrylates, poly(meth)acrylic esters and/or crosslinked products thereof, and rubbers such as ethylene rubbers, propylene rubbers, styrene-butadiene rubbers, butadiene rubbers and silicone rubbers and/or crosslinked products thereof; and natural polymers and/or derivatives thereof such as cellulose and/or derivatives thereof, chitosan and/or derivatives thereof, starch and shells of fruits. Of these, polyethylenes, polyamides, polystyrenes, sodium (poly)methacrylates, poly(meth)acrylic esters, cellulose and/or derivatives thereof, and starches are more preferred. The term "poly(meth)acrylate" as used herein means both "polyacrylate" and "polymethacrylate".

Preferred examples of the water-insoluble inorganic primary particles include bentonite, talc, mica, kaolin, sepiolite, silica, calcium carbonate, titanium oxide, silicic anhydride, hydroxy.calcium.apatite, and mother of pearl. Of these, bentonite, talc, mica, kaolin and silica are more preferred.

These water-insoluble primary particles may be in the spherical form, substantially spherical form, or other forms obtained by pulverization or the like. In addition, hollow or porous particles may also be employed. These water-insoluble primary particles may be used either singly or in combination of two or more.

Examples of the water-soluble organic primary particles include synthetic polymers such as polyvinyl alcohols and/or derivatives thereof, alkali salts of a poly(meth)acrylic acid, alkali salts of a (meth)acrylic acid/(meth)acrylate ester copolymer, alkali salts of an acrylic acid/maleic acid copolymer, and polyvinylpyrrolidones; saccharides such as methyl cellulose, ethyl cellulose, sodium carboxymethyl cellulose, hydroxyalkyl cellulose, modified starches (e.g., hydroxyalkyl-modified starches, and phosphate-modified starches), sucrose and lactose; natural polymers such as seaweeds and proteins.

Examples of the water-soluble inorganic primary particles include chlorides such as sodium chloride, potassium chloride and magnesium chloride; sulfates such as sodium sulfate, potassium sulfate, magnesium sulfate and aluminum sulfate; and carbonates such as sodium carbonate and sodium bicarbonate. As the sodium chloride, a commercially available salt, a highly purified salt and a natural salt are employed. Of these, inorganic particles such as sodium chloride, potassium chloride, magnesium chloride and sodium carbonate are preferred.

No particular limitation is imposed on the form of these water-soluble primary particles and they may be used either singly or in combination of two or more.

In the disintegrating particles of the present invention, the weight ratio of the water-insoluble primary particles to the water-soluble primary particles, (water insoluble-primary particles)/(water-soluble primary particles), preferably ranges from 1/99 to 100/0.

These primary particles have preferably an average particle size of not more than 50 µm because the primary particles having an average particle size within the above-described range do not leave a strange feel and can be rinsed off well when the disintegrating particles disintegrate during a cleansing step or by the action of rinse water or tears.

The average particle size of the disintegrating particles of the present invention is preferably not less than 30 µm but less than 100 µm, more preferably not less than 50 µm but not more than 98 µm, even more preferably not less than 50 µm but not more than 95 µm. As the average particle size, a median size as determined using a laser diffraction/scattering particle size distribution analyzer "LA-910" (product of Horiba, Ltd.) is adopted.

The disintegrating particles of the present invention are obtained by binding and agglomerating the above-described primary particles with a water-soluble binder. Although there is no particular limitation is imposed on such a water-soluble binder insofar as it dissolves in an aqueous solution of a water-soluble salt when the concentration of the salt lowers and precipitates when the concentration of the salt rises. Examples of the water-soluble binder include synthetic products such as polyvinyl alcohols and/or derivatives thereof (itaconic acid-modified polyvinyl alcohols, sulfonic acid-modified polyvinyl alcohols, maleic acid-modified polyvinyl alcohols and the like), alkali salts of a poly(meth)acrylic acid, alkali salts of a (meth)acrylic acid/(meth)acrylate copolymer, alkali salts of an acrylic acid/maleic acid copolymer, and polyvinylpyrrolidones; semi-synthetic polymers such as methyl cellulose, ethyl cellulose, sodium carboxymethyl cellulose, hydroxyalkyl cellulose and starch derivatives; natural polymers such as starches, seaweeds, plant viscous substances and proteins.

In the disintegrating particles of the present invention, the water-insoluble primary particles and the water-soluble binder may be composed of the same or different materials.

It is preferred from the standpoints of easy disintegration of the particles and workability during preparation of the desintegrating particles or a detergent composition containing them that the water-soluble binder is added in an amount of from 0.5 to 30 wt.% based on the weight of the primary particles.

Although no particular limitation is imposed on the process for preparation of the disintegrating particles of the present invention, it is preferred, for example, to mix the primary particles with the water-soluble binder and then granulate the resulting mixture by a granulation method such as rolling granulation, rolling fluidized-bed granulation, fluidized-bed granulation, stirring rolling granulation, melting granulation, extrusion granulation, spray drying granulation and/or coating method such as spray drying; and/or to use the above-described method while mixing them.

The disintegrating particles of the present invention thus prepared are as above characterized in that their disintegration ratio increases as the concentration of a water-soluble salt in an aqueous solution containing the water-soluble salt lowers. When these disintegrating particles are incorporated in a detergent composition, they are stably dispersed in the detergent composition without causing disintegration but disintegrate as the concentration of the water-soluble salt lowers during cleansing or rinsing step. When such incorporation in a detergent composition is taken into consideration, it is preferred to design the disintegrating characteristic of the disintegrating particles of the present invention in such a manner that at least a part of them disintegrates in a water-solution having a water-soluble salt concentration of less than 1.0 wt.%, more preferably less than 1.5 wt.%. From the standpoints of rinse-off with rinse water or tears, it is more preferred to design it in such a manner that not less than 60 vol.% of the particles disintegrates in an aqueous solution having a water-soluble salt concentration of less than 1.0 wt.%, more preferably less than 1.5 wt.%. The particles are then broken into particles having an average particle size of preferably not more than 50 µm, more preferably not more than 30 µm.

The disintegration ratio of the disintegrating particles is measured as described below. After 0.3 g of disintegrating particles is added to 29.7 g of purified water or an aqueous salt solution having a concentration of the salt less than 1.5 wt.%, they are stored in a thermostat at 35°C for 15 hours. Then, 6 g of the sample are weighed on an artificial leather and the leather is massaged by one hand for 1 second (once reciprocating back and fourth). The particle size of the sample is then measured by a laser diffraction/scattering particle size distribution analyzer "LA-910" (Horiba, Ltd.). The ratio of the volume of the disintegrating particles broken into particles of not more than 30 µm is expressed as a volume percentage and the percentage is referred to as a disintegration ratio (%).

The detergent composition of the present invention contains the above-described disintegrating particles, a water-soluble salt, a surfactant and water, and the concentration of the water-soluble salt is less than saturation solubility (solubility at saturation point).

The amount of the disintegrating particles in the detergent composition of the present invention is preferably from 1 to 25 wt.%, more preferably from 2 to 20 wt.% from the standpoints of touch feel and physical (mechanical) detergency.

The water-soluble salt to be added as Component (B) to the detergent composition of the present invention include water-soluble inorganic salts and water-soluble organic salts. Of these, water-soluble inorganic salts are preferred.

The water-soluble inorganic salt include chlorides such as sodium chloride, potassium chloride and magnesium chloride; sulfates such as sodium sulfate, potassium sulfate, magnesium sulfate and aluminum sulfate; and carbonates such as sodium carbonate and sodium bicarbonate. As the sodium chloride, commercially available salts, highly purified salts and natural salts are used. Of these, sodium chloride, potassium chloride, magnesium chloride and sodium carbonate are preferred.

The water-soluble organic salt include salts of citric acid, succinic acid, maleic acid, fumaric acid and malic acid, as well as anionic surfactants such as fatty acid soaps, ester phosphates, acylated amino acid salts, sulfosuccinates, and taurate surfactants. The water-soluble inorganic salt and water-soluble organic salt can be used in combination. In this case, they are used preferably at a (water-soluble inorganic salt)/(water soluble-organic salt) weight ratio ranging from 100/0 to 5/95.

The water-soluble salt is added in an amount less than the amount at saturation solubility in water in the detergent composition. From the viewpoints of stability of the disintegrating particles in the composition, disintegration of the particles by rinsing or tears, and foaming property of the detergent, the amount is not less than 1.5 wt.% but less than saturation solubility, still more preferably not less than 2 wt.% but not more than 0.8 time the weight of the saturation solubility.

Although no particular limitation is imposed on the surfactant as Component (C) of the present invention, anionic surfactants, nonionic surfactants, and amphoteric surfactants can be used. Examples of the anionic surfactants include phosphates, fatty acid salts, alkyl sulfates, polyoxyalkylene alkyl ether sulfates, sulfosuccinic acid surfactants, polyoxyalkylene alkylamidoether sulfates, monoglyceride sulfates, olefin sulfonates, alkanesulfonates, acylated isethionates, acylated amino acid salts, and polyoxyalkylene alkyl ether phosphates. Examples of the nonionic surfactants include alkyl polyglycosides such as alkyl polyglucosides, sucrose fatty acid esters, polyglycerin fatty acid esters, polyoxyalkylene alkyl ethers, fatty acid alkanolamides, alkylamine oxides, and fatty acid polyol esters. Examples of the amphoteric surfactant include amide amino acid surfactants, carbobetaine surfactants, sulfobetaine surfactants, amidosulfobetaine surfactants, imidazolinium betaine surfactants, amino acid betaine surfactants, and phosphobetaine surfactants. Of these, phosphates, acylated amino acids, alkyl saccharides are preferred, with phosphates being more preferred because of low irritation to the skin. These surfactants may be used either singly or in combination of two or more. They are added to a skin detergent preferably in an amount of from 5 to 95 wt.%.

The phosphate to be used as Component (C) in the present invention is preferably a phosphate surfactant which is a mixture of (c1) a phosphate monoester represented by formula (1) and (c2) a phosphate diester represented by formula (2) and contains them at a (c1)/(c2) weight ratio ranging from 100/0 to 50/50. 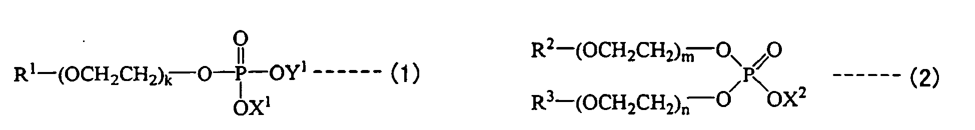 (wherein, R¹, R² and R³ each represents a linear or branched alkyl or alkenyl group having 8 to 18 carbon atoms, X¹, X² and Y¹ each represents a hydrogen atom, an alkali metal atom, an alkaline earth metal atom, an alkanolamine or ammonium, and k, m and n each represents an average number of the moles added and is from 0 to 10). In formulas (1) and (2), R¹, R² and R³ each represents a linear or branched alkyl or alkenyl group having 8 to 18 carbon atoms, preferably a linear or branched alkyl or alkenyl group having 10 to 16 carbon atoms.

R¹ in formula (1) is preferably a linear alkyl or alkenyl group having 8 to 18 carbon atoms, with a linear alkyl group such as octyl, nonyl, decyl, undecyl, dodecyl, tridecyl, tetradecyl or pentadecyl being more preferred.

R² and R³ in formula (2) are each preferably a linear alkyl or alkenyl group having 8 to 18 carbon atoms, with a linear alkyl group such as octyl, nonyl, decyl, undecyl, dodecyl, tridecyl, tetradecyl or pentadecyl being more preferred.

As X¹, X² and X³ in formulas (1) and (2), examples of the alkali metal include lithium, sodium and potassium; those of the alkaline earth metal include magnesium; and those of the alkanolamine include triethanolamine, diethanolamine and monoethanolamine. Of these, alkali metals such as potassium and sodium and triethanolamine are preferred, with potassium being more preferred from the viewpoint of solubility in water.

Letters "k", "m" and "n" each represents an average number of moles added and is preferably a number of from 0 to 4, more preferably from 0 to 2.

Components (c1) and (c2) in the phosphate of Component (C) are added preferably at a (c1)/(c2) weight ratio ranging from 100/0 to 50/50, more preferably from 100/0 to 60/40, still more preferably from 100/0 to 70/30 from the standpoint of foaming property. From the standpoints of foaming property and detergency, a higher content of (c1) is preferred.

Component (C) of the detergent composition of the present invention is incorporated in the whole composition preferably in an amount of from 1 to 50 wt.%, more preferably from 3 to 30 wt.%, still more preferably from 5 to 20 wt.% from the standpoints of foaming property and detergency.

The detergent composition of the present invention may further comprise (E) a glyceryl ether. The glyceryl ether to be used as Component (E) in the present invention has a linear or branched alkyl or alkenyl group having 4 to 12 carbon atoms. Glyceryl ethers having an alkyl group having 4 to 12 carbon atoms, for example, n-butyl, isobutyl, tert-butyl, n-pentyl, isopentyl, n-hexyl, isohexyl, n-heptyl, n-octyl, 2-ethylhexyl, n-nonyl, n-decyl or n-lauryl are preferred, of which those having 4 to 11 carbon atoms are more preferred and those having 6 to 10 carbon atoms is still more preferred. Of these, those having one or two alkyl groups, especially one alkyl group, of 8 carbon atoms is preferred.

As Component (E), in order to improve fluidity and foaming property, one or more glyceryl ethers can be used. It is incorporated preferably in an amount of from 0.1 to 20 wt.%, more preferably from 0.1 to 10 wt.%, still more preferably from 0.1 to 5 wt.% in the whole composition from the viewpoints of fluidity and foaming property.

The detergent composition of the present invention may further comprise, as Component (F),in order to improve fluidity and foaming property, a compound represented by the following formula (3):

R⁹O-(AO)ₚ-R⁵ (3)

In the alkylene oxide adduct of the alcohol represented by formula (3) and serving as Component (F), R⁴ represents a linear or branched alkyl or alkenyl group having 6 to 10 carbon atoms, preferably 8 to 10 carbon atoms, with an alkyl group having 8 carbon atoms being more preferred. AO represents an alkyleneoxy group having 2 to 4 carbon atoms, preferably an alkyleneoxy group having 2 or 3 carbon atoms, more preferably an alkyleneoxy group having 3 carbon atoms. Specific examples include ethyleneoxy, propyleneoxy and butyleneoxy groups, of which ethyleneoxy and propyleneoxy groups are preferred and propyleneoxy group is more preferred. The letter "p" represents an average number of moles added and is from 0.5 to 3, preferably from 1 to 3, more preferably from 2 to 3. R⁵ represents a hydrogen atom or methyl group, with a hydrogen atom being preferred.

As Component (F), one or more of the above-described compounds may be used. It is incorporated preferably in an amount of from 0.1 to 20 wt.%, more preferably from 0.1 to 15 wt.%, still more preferably from 0.1 to 10 wt.% in the whole composition from the viewpoints of fluidity and foaming property.

Component (A) and Component (B) are incorporated in the detergent composition of the present invention preferably at an (A)/(B) weight ratio ranging from 1/10 to 20/1, more preferably from 1/5 to 10/1, still more preferably from 1/1 to 7/1 from the standpoints of detergency and feeling upon use.

Component (A) and Component (C) are incorporated in the detergent composition of the present invention preferably at an (A)/(C) weight ratio ranging from 1/10 to 6/1, more preferably from 1/5 to 5/1, still more preferably from 1/1 to 4/1 from the standpoints of detergency and feeling upon use.

Component (A) and Component (E) are incorporated in the detergent composition of the present invention preferably at an (A)/(E) weight ratio ranging from 3/1 to 40/1, more preferably from 5/1 to 35/1, still more preferably from 10/1 to 30/1 from the standpoints of detergency and feeling upon use.

Component (A) and Component (F) are incorporated in the detergent composition of the present invention preferably at an (A)/(F) weight ratio ranging from 3/1 to 30/1, more preferably from 5/1 to 20/1, still more preferably from 10/1 to 15/1 from the standpoints of detergency and feeling upon use.

It is preferable that a 20-fold dilution of the detergent composition has a pH of from 4 to 8.

The detergent composition is preferably used together with water having a hardness of from 1 to 30°DH.

The water used preferably has a pH of from 4 to 9.

In addition to the above-described components, components conventionally employed for detergents such as oily substances, thickeners, humectants, colorants, antiseptics, feel improvers, perfumes, anti-inflammatory agents, bactericides, and ultraviolet absorbers may be added within an extent not impairing the advantages of the present invention.

The detergent composition of the present invention can be used widely for, for example, detergents for skin such as face wash, body shampoo and solid soap, shampoos, detergents for dish, detergents for contact lens and toothpastes and moreover, for massage creams.

The detergent composition of the present invention is preferably used for cleansing a body.

### [Examples]

### Examples 1 to 27 and Comparative Examples 1 to 6

Detergent compositions were prepared by mixing the components as shown in Tables 1 to 3 and adjusted to pH 5 to 6. They were evaluated for foaming property, massage effect, foreign-body sensation during massage, detergency and rinse-off property in accordance with the below-described criteria. The results are shown in Tables 1 to 3. The average particle size was measured by a laser diffraction/scattering particle size distribution analyzer "LA-910" (product of Horiba, Ltd.). A median size was adopted as the average particle size.

### (Evaluation method 1: ordinary cleansing)

### Foaming property, massage effect, foreign-body sensation during massage, detergency and rinse-off property

A panel of 10 experts applied 2 ml of a detergent composition, which had been prepared in a manner known per se in the art, to their palm and cleansed their face freely. They evaluated the foaming property, massage effects, foreign-body sensation during massage, detergency and rinse-off property at that time in accordance with the below-described criteria.

### (Evaluation method 2: high concentration cleansing (cleansing manner in Asia))

### Foaming property, massage effect, foreign-body sensation during massage, detergency and rinse-off property

A panel of 10 experts applied 1 ml of a detergent composition, which had been prepared in a manner known per se in the art, to their palm and cleansed their face without foaming while using 2 mL of hard water having a hardness of 15°DH. They evaluated the foaming property, massage effects, foreign-body sensation during massage, detergency and rinse-off property at that time in accordance with the below-described criteria.

### Evaluation criteria

### (Foaming property)

A: 8 to 10 experts judged the composition to be good
B: 6 to 7 experts judged the composition to be good
C: 5 or less experts judged the composition to be good
D: at least 1 expert judged the composition to be insufficient

### (Massage effect)

A: 8 to 10 experts judged the composition to be good
B: 6 to 7 experts judged the composition to be good
C: 5 or less experts judged the composition to be good
D: at least 1 expert felt no massage effect

### (Foreign-body sensation during massage)

A: 8 to 10 experts felt no such sensation
B: 6 to 7 experts felt no such sensation
C: 5 or less experts felt no such sensation
D: at least 1 expert felt discomfort or such sensation

### (Detergency)

A: 8 to 10 experts judged the composition to be good
B: 6 to 7 experts judged the composition to be good
C: 5 or less experts judged the composition to be good
D: at least 1 expert judged the composition to be insufficient

### (Rinse-off property)

A: 8 to 10 experts judged the composition to be good
B: 6 to 7 experts judged the composition to be good
C: 5 or less experts judged the composition to be good
D: at least 1 expert felt that the composition was not rinsed off completely.

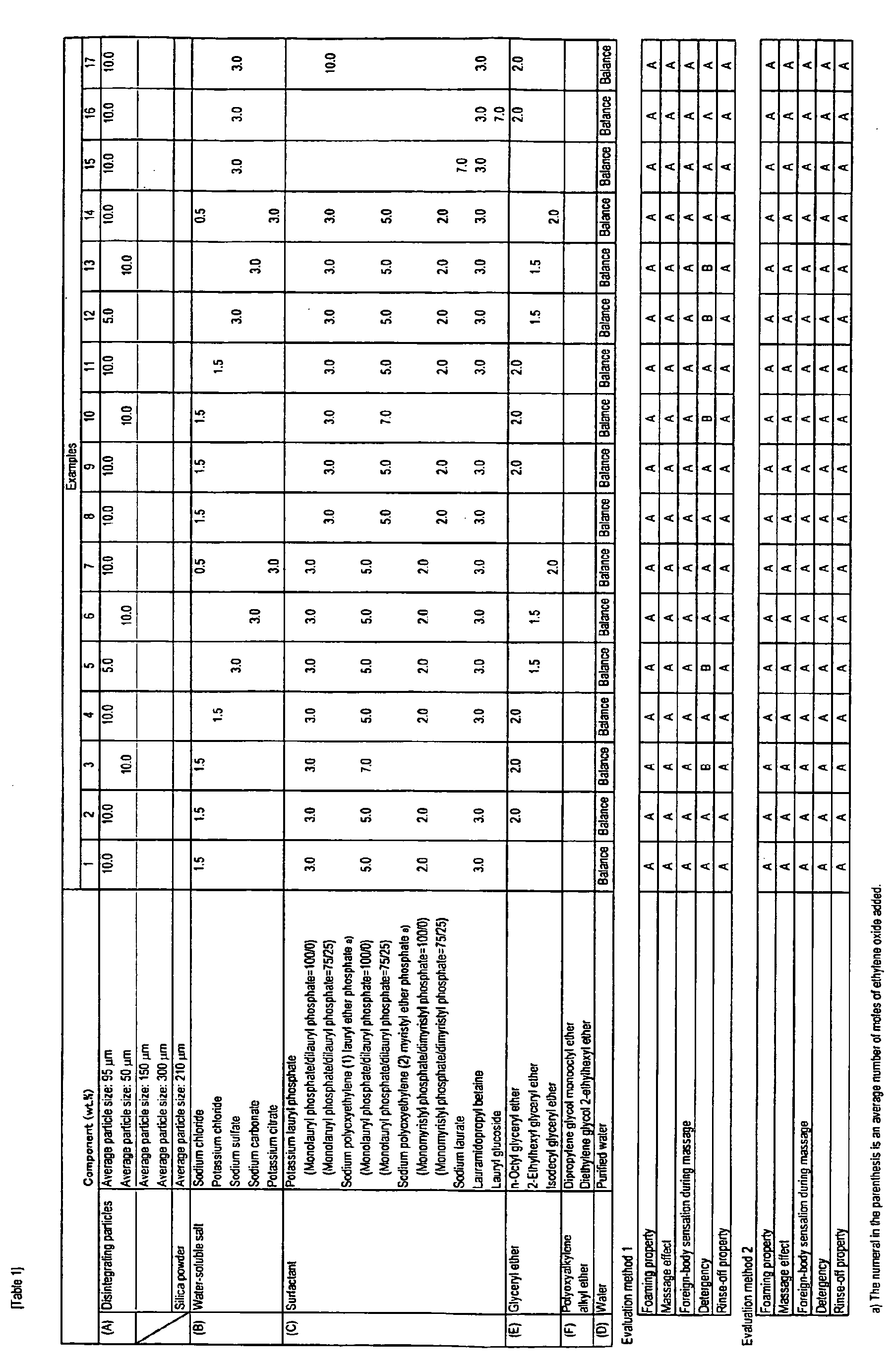

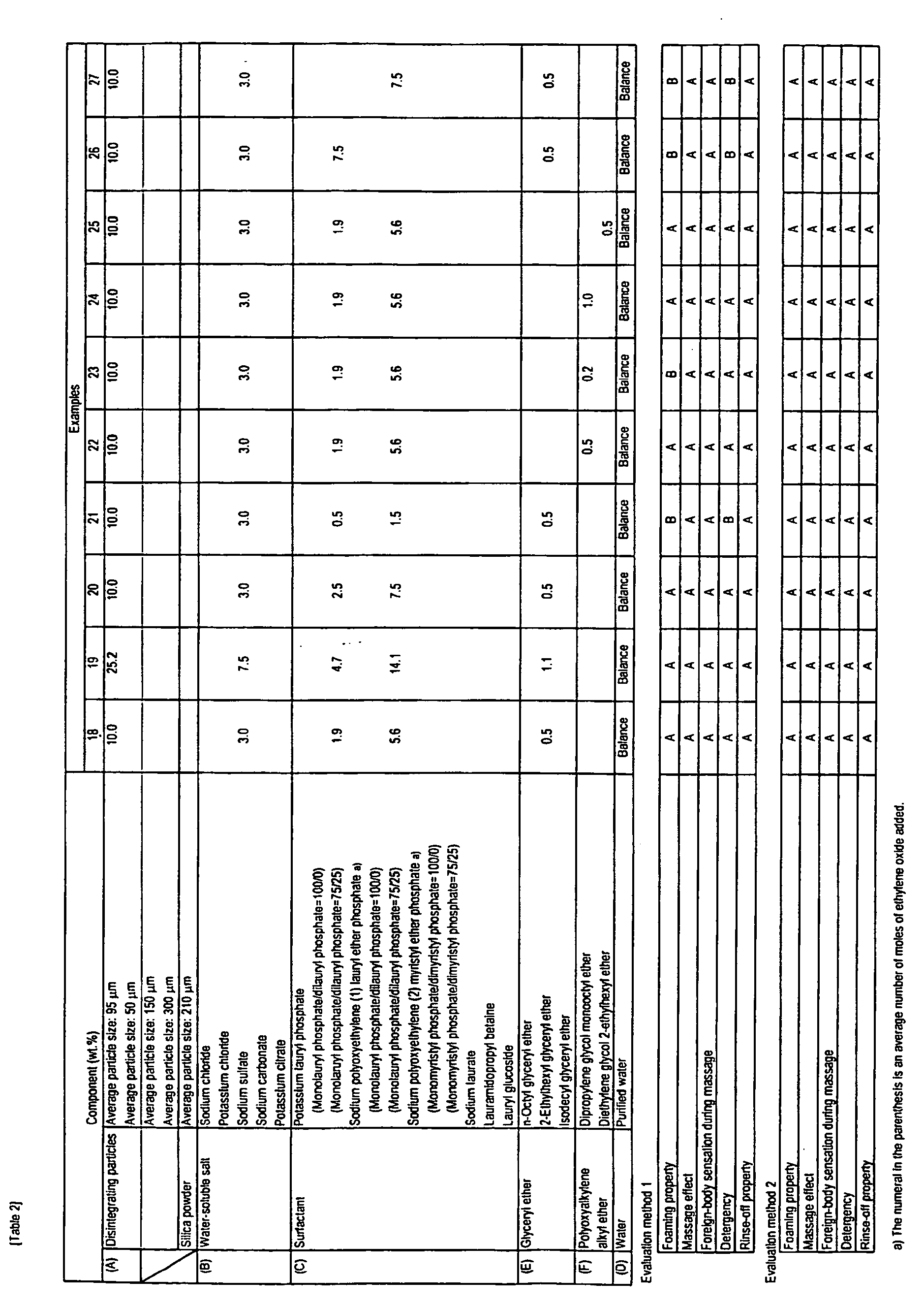

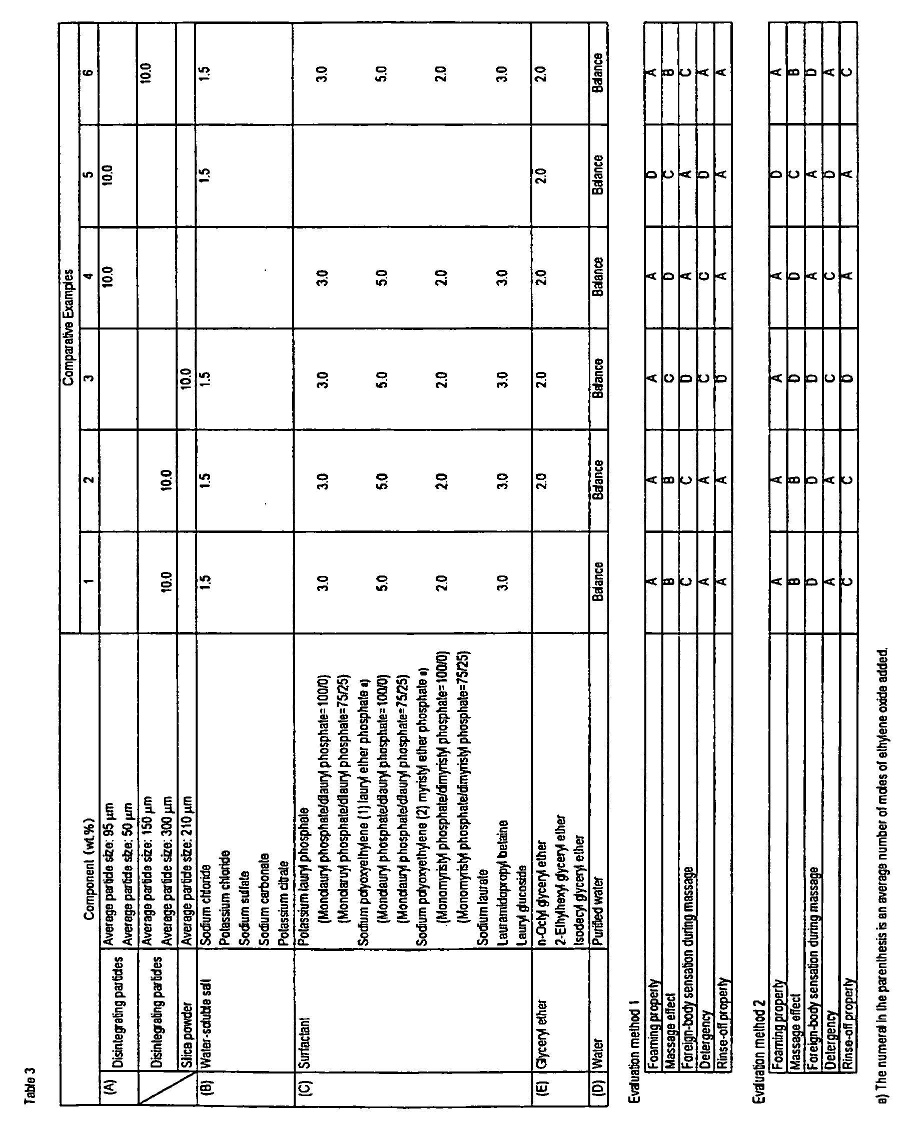

### Examples 28 to 38

Face washes, body shampoos and shampoos, each having a pH of from 5.0 to 6.0 were prepared in accordance with the below-described formulations. The pH was adjusted by citric acid or potassium hydroxide. Any of them were low irritating, left no foreign-body sensation during massage, and exhibited excellent time-dependent stability, foaming property and detergency in any of pH regions of from 4 to less than 5, from 5 to less than 8 and from 8 to 9.

### Example 28 (face wash)

| (Component) | (parts by weight) |
|---|---|
| Potassium lauryl phosphate (monolauryl phosphate/dilauryl phosphate= 100/0) | 2.00 |
| Potassium polyoxyethylene (1) lauryl ether phosphate^{a)} (monolauryl phosphate/dilauryl phosphate = 100/0) | 6.00 |
| 2-Ethylhexyl glyceryl ether | 1.30 |
| Dipropylene glycol | 1.20 |
| Lauramidopropyl betaine | 1.00 |
| Polyoxyethylene sorbitan triisostearate (160EO) | 0.50 |
| Acrylic acid/alkyl methacrylate copolymer ^{b)} | 0.20 |
| A 4 wt.% aqueous solution of N,N-dimethylaminoethyl methacrylate diethyl sulfate-N,N-dimethylacrylamide-polyethylene glycol dimethacrylate copolymer ^{c)} | 2.50 |
| Disintegrating particles (average particle size: 95 µm) | 12.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Carbopol ETD2020", product of BF Goodrich c): "Sofcare KG-301W", product of Kao Corp. | |

### Example 29 (Face wash)

| (Component) | (parts by weight) |
|---|---|
| Potassium lauryl phosphate (monolauryl phosphate/dilauryl phosphoate = 75/25) | 2.00 |
| Potassium polyoxyethylene (1) lauryl ether phosphate^{a)} (monolauryl phosphate/dilauryl phosphate = 75/25) | 6.00 |
| 2-Ethylhexyl glyceryl ether | 1.30 |
| Dipropylene glycol | 1.20 |
| Lauramidopropyl betaine | 1.00 |
| Polyoxyethylene sorbitan triisostearate (160EO) | 0.50 |
| Acrylic acid/alkyl methacrylate copolymer ^{b)} | 0.20 |
| A 4 wt.% aqueous solution of N,N-dimethylaminoethyl methacrylate diethyl sulfate-N,N-dimethylacrylamide-polyethylene glycol dimethacrylate copolymer ^{c)} | 2.50 |
| Disintegrating particles (average particle size: 95 µm) | 12.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Carbopol ETD2020", product of BF Goodrich c): "Sofcare KG-301W", product of Kao Corp. | |

### Example 30 (Body shampoo)

| (Component) | (parts by weight) |
|---|---|
| Potassium lauryl phosphate (monolauryl phosphate/dilauryl phosphate=100/0) | 2.50 |
| Potassium polyoxyethylene (1) lauryl ether phosphate^{a)} (monolaurylphosphate/dilaurylphosphate = 100/0) | 7.50 |
| 2-Ethylhexyl glyceryl ether | 2.30 |
| Dipropylene glycol | 1.70 |
| Lauramidopropyl betaine | 3.00 |
| Polyoxyethylene sorbitan triisostearate (160EO) | 0.50 |
| A 4 wt.% aqueous solution of N,N-dimethylaminoethyl methacrylate diethyl sulfate-N,N-dimethylacrylamide-polyethylene glycol dimethacrylate copolymer ^{c)} | 2.50 |
| Disintegrating particles (average particle size: 95 µm) | 10.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Sofcare KG-301W", product of Kao Corp. | |

### Example 31 (Body shampoo)

| (Component) | (parts by weight) |
|---|---|
| Potassium lauryl phosphate (monolauryl phosphate/dilauryl phosphate=75/25) | 2.50 |
| Potassium polyoxyethylene (1) lauryl ether phosphate^{a)} (monolauryl phosphate/dilauryl phosphate = 75/25) | 7.50 |
| 2-Ethylhexyl glyceryl ether | 2.30 |
| Dipropylene glycol | 1.70 |
| Lauramidopropyl betaine | 3.00 |
| Polyoxyethylene sorbitan triisostearate (160EO) | 0.50 |
| A 4 wt.% aqueous solution of N,N-dimethylaminoethyl methacrylate diethyl sulfate-N,N-dimethylacrylamide-polyethylene glycol dimethacrylate copolymer ^{b)} | 5.00 |
| Disintegrating particles (average particle size: 95 µm) | 10.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Sofcare KG-301W", product of Kao Corp. | |

### Example 32 (Shampoo)

| (Component) | (parts by weight) |
|---|---|
| Sodium polyoxyethylene (2) lauryl ether sulfate ^{a)} | 15.00 |
| 2-Ethylhexyl glyceryl ether | 2.00 |
| Dipropylene glycol | 1.30 |
| Lauramidopropyl betaine | 3.00 |
| A 4 wt.% aqueous solution of N,N-dimethylaminoethyl methacrylate diethyl sulfate-N,N-dimethylacrylamide-polyethylene glycol dimethacrylate copolymer ^{b)} | 5.00 |
| Disintegrating particles (average particle size: 95 µm) | 5.00 |
| Sodium chloride | 3.00 |
| Ethylene glycol distearate | 1.00 |
| Methylparaben | 0.20 |
| Perfume | 0.05 |
| Purified water | Balance |
| Total | 100.0 |

| | |
|---|---|
| a): The numeral in the parenthesis is an average number of moles of ethylene oxide added. b): "Sofcare KG-301W", product of Kao Corp | |

### Example 33 (Shampoo)

| (Component) | (parts by weight) |
|---|---|
| Sodium polyoxyethylene (2) lauryl ether sulfate ^{a)} | 15.00 |
| 2-Ethylhexyl glyceryl ether | 2.00 |
| Dipropylene glycol | 1.30 |
| Lauramidopropyl betaine | 3.00 |
| A 4 wt.% aqueous solution of N,N-dimethylaminoethyl methacrylate diethyl sulfate-N,N-dimethylacrylamide-polyethylene glycol dimethacrylate copolymer ^{b)} | 5.00 |
| Disintegrating particles (average particle size: 95 µm) | 5.00 |
| Sodium chloride | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Sofcare KG-301W", product of Kao Corp | |

### Example 34 (Shampoo)

| (Component) | (parts by weight) |
|---|---|
| Sodium polyoxyethylene (2) lauryl ether sulfate ^{a)} | 15.00 |
| Isodecyl glyceryll ether | 2.00 |
| Dipropylene glycol | 1.30 |
| Lauramidopropyl betaine | 3.00 |
| A 4 wt.% aqueous solution of N,N-dimethylaminoethyl methacrylate diethyl sulfate-N,N-dimethylacrylamide-polyethylene glycol dimethacrylate copolymer ^{b)} | 5.00 |
| Disintegrating particles (average particle size: 95 µm) | 5.00 |
| Sodium chloride | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Sofcare KG-301W", product of Kao Corp | |

### Example 35 (Shampoo)

| (Component) | (parts by weight) |
|---|---|
| Sodium polyoxyethylene (2) lauryl ether sulfate ^{a)} | 12.00 |
| Potassium lauryl phosphate (monolauryl phosphate/dilauryl phosphate = 75/25) | 1.00 |
| Potassium polyoxyethylene (1) lauryl ether phosphate^{a)} (monolauryl phosphate/dilauryl phosphate = 75/25) | 3.00 |
| 2-Ethylhexyl glyceryl ether | 0.24 |
| Dipropylene glycol | 0.68 |
| Lauramidopropyl betaine | 3.00 |
| Cationized cellulose ^{b)} | 0.30 |
| Disintegrating particles (average particle size: 95 µm) | 10.00 |
| Sodium chloride | 1.50 |
| Citric acid | 0.10 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Poiz C-80M", product of Kao Corp. | |

### Example 36 (face wash)

| | (parts by weight) |
|---|---|
| Potassium lauryl phosphate (monolauryl phosphate/dilauryl phosphate = 75/25) | 2.00 |
| Potassium polyoxyethylene (1) lauryl ether phosphate^{a)} (monolauryl phosphate/dilauryl phosphate = 75/25) | 6.00 |
| Dipropylene glycol monooctyl ether | 1.30 |
| Dipropylene glycol | 1.20 |
| Lauramidopropyl betaine | 1.00 |
| Polyoxyethylene sorbitan triisostearate (160EO) | 0.50 |
| Acrylic acid/alkyl methacrylate copolymer ^{b)} | 0.20 |
| A 4 wt.% aqueous solution of N,N-dimethylaminoethyl methacrylate diethyl sulfate-N,N-dimethylacrylamide-polyethylene glycol dimethacrylate copolymer ^{c)} | 2.50 |
| Disintegrating particles (average particle size: 95 µm) | 12.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Carbopol ETD2020", product of BF Goodrich c): "Sofcare KG-301W", product of Kao Corp. | |

### Example 37 (body shampoo)

| | (parts by weight) |
|---|---|
| Potassium lauryl phosphate (monolauryl phosphate/dilauryl phosphate = 75/25) | 2.50 |
| Potassium polyoxyethylene (1) lauryl ether phosphate^{a)} (monolauryl phosphate/dilauryl phosphate = 75/25) | 7.50 |
| Dipropylene glycol monooctyl ether | 2.30 |
| Dipropylene glycol | 1.70 |
| Lauramidopropyl betaine | 3.00 |
| Polyoxyethylene sorbitan triisostearate (160EO) | 0.50 |
| A 4 wt.% aqueous solution of N,N-dimethylaminoethyl methacrylate diethyl sulfate-N,N-dimethylacrylamide-polyethylene glycol dimethacrylate copolymer ^{b)} | 5.00 |
| Disintegrating particles (average particle size: 95 µm) | 10.00 |
| Sodium sulfate | 3.00 |
| Ethylene glycol distearate | 1.00 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Sofcare KG-301W", product of Kao Corp. | |

### Example 38 (shampoo)

| | (parts by weight) |
|---|---|
| Sodium polyoxyethylene (2) lauryl ether sulfate ^{a)} | 12.00 |
| Potassium lauryl phosphate (monolauryl phosphate/dilauryl phosphate = 75/25) | 1.00 |
| Potassium polyoxyethylene (1) lauryl ether phosphate^{a)} (monolauryl phosphate/dilauryl phosphate = 75/25) | 3.00 |
| Dipropylene glycol monooctyl ether | 0.24 |
| Dipropylene glycol | 0.68 |
| Lauramidopropyl betaine | 3.00 |
| Cationized cellulose ^{b)} | 0.30 |
| Disintegrating particles (average particle size: 95 µm) | 10.00 |
| Sodium chloride | 1.50 |
| Citric acid | 0.10 |
| Methylparaben | 0.20 |
| Perfume | 0.05 |
| Purified water | Balance |
| Total | 100.00 |

| | |
|---|---|
| a): The numeral in the parenthesis is an average number of moles of ethylene oxide added. b): "Poiz C-80M", product of Kao Corp. | |

## Claims

1. A detergent composition, comprising the following components (A), (B), (C) and (D):
(A) disintegrating particles for detergent obtained by binding and agglomerating primary particles at least a part of which is water-insoluble with a water-soluble binder and having an average particle size of not less than 30 µm but less than 100 µm,
(B) a water-soluble salt selected from inorganic salts of chlorides, sulfates and carbonates, organic salts of citric acid, succinic acid, maleic acid, fumaric acid and malic acid, and anionic surfactants, wherein the water-soluble salt is contained in an amount of not less than 1.5 wt.%, but less than saturation solubility
(C) a surfactant except glyceryl ether, and
(D) water,
wherein the term "water-insoluble" means that the solubility of the particles at 25°C is less than 0.5 g in 100 g of water,
wherein the term "water-soluble" means that the solubility of the particles at 25°C is not less than 0.5 g in 100 g water, and
wherein the average particle size is the median size as determined using a laser diffraction/scattering particles size distribution analyzer.

2. The detergent composition according to Claim 1, wherein the disintegrating particles disintegrate as the concentration of the water-soluble salt in a water-soluble salt-containing aqueous solution lowers.

3. The detergent composition according to Claim 1 or 2, wherein Component (C) is a phosphate surfactant which is a mixture of (c1) a phosphate monoester represented by formula (1) and (c2) a phosphate diester represented by formula (2) and Components (c1) und (c2) are contained at (c1)/(c2) weight ratio falling within a range of from 100/0 to 50/50: 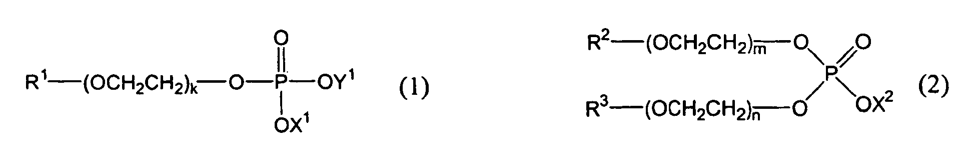 wherein,R¹, R² and R³ each represent a linear or branched alkyl or alkenyl group having 8 to 18 carbon atoms, X¹, X² and Y¹ each represents a hydrogen atom, an alkali metal atom, an alkaline earth metal atom, an alkanolamine or ammonium, and k, m and n each represents an average number of moles added and is from 0 to 10.

4. The detergent composition according to any one of Claims 1 to 3, further comprising, as Component (E), at least one glyceryl ether having an alkyl or alkenyl group having 4 to 12 carbon atoms.

5. The detergent composition according to any one of Claims 1 to 4, further comprising, as Component (F), a compound represented by the following formula (3):
R⁴O-(AO)ₚ-R⁵ (3)
wherein, R⁴ represents a linear or branched alkyl or alkenyl group having 6 to 10 carbon atoms, R⁵ represents a hydrogen atom or methyl group, AO represents an alkyleneoxy group having 2 to 4 carbon atoms and p represents an average number of moles added and is a number of 0.5 to 3.

6. The detergent composition according to any one of Claims 1 to 5, wherein a 20-fold dilution of the detergent composition has a pH of from 4 to 8.

7. The detergent composition according to any one of Claims 1 to 6, which is used together with water having a hardness of from 1 to 30°DH.

8. The detergent composition according to any one of Claims 1 to 7, wherein the water used has a pH of from 4 to 9.

9. The detergent composition according to Claim 4, wherein the content of Component (E) is from 0.3 to 5 wt.%.

10. The detergent composition according to Claim 5, wherein the content of Component (F) is from 0.3 to 10 wt.%.

11. A method for cleansing a body, which comprises applying the detergent composition as claimed in any one of Claims 1 to 10 to the body.

12. Use of the detergent composition as claimed in any one of Claims 1 to 10 for cleansing a body.

## Patentansprüche

1. Reinigungszusammensetzung, umfassend die folgenden Komponenten (A), (B), (C) und (D) :
(A) sich auflösende Teilchen für ein Reinigungsmittel, erhalten durch Binden und Agglomerieren von Primärteilchen, wobei zumindest ein Teil davon wasserunlöslich ist, mit einem wasserlöslichen Bindemittel und mit einer durchschnittlichen Teilchengröße von nicht weniger als 30 µm, aber weniger als 100 µm,
(B) ein wasserlösliches Salz, ausgewählt aus anorganischen Salzen von Chloriden, Sulfaten und Carbonaten, organischen Salzen von Zitronensäure, Succinsäure, Maleinsäure, Fumarsäure und Äpfelsäure, und anionischen Tensiden, worin das wasserlösliche Salz in einer Menge von nicht weniger als 1,5 Gew.-%, aber weniger als der Sättigungslöslichkeit enthalten ist,
(C) ein Tensid (mit Ausnahme von Glycerylether) und
(D) Wasser,
worin der Ausdruck "wasserunlöslich" bedeutet, dass die Löslichkeit der Teilchen bei 25°C weniger als 0,5 g in 100 g Wasser ist,
worin der Ausdruck "wasserlöslich" bedeutet, dass die Löslichkeit der Teilchen bei 25°C nicht weniger als 0,5 g in 100 g Wasser ist, und
worin die durchschnittliche Teilchengröße die mittlere Größe ist, bestimmt unter Verwendung eines Laserbeugungs/Streuungsanalysegerätes für die Teilchengrößenverteilung.

2. Reinigungszusammensetzung nach Anspruch 1, worin die sich auflösenden Teilchen sich auflösen, wenn sich die Konzentration des wasserlöslichen Salzes in einer wässrigen Lösung, die wasserlösliche Salze enthält, erniedrigt.

3. Reinigungszusammensetzung nach Anspruch 1 oder 2, worin die Komponente (C) ein Phosphattensid ist, das eine Mischung aus (c1) einem Phosphatmonoester mit der Formel (1) und (c2) einem Phosphatdiester mit der Formel (2) ist und die Komponenten (c1) und (c2) in einem (c1)/(c2) Gewichtsverhältnis enthalten sind, das innerhalb eines Bereiches von 100/0 bis 50:50 fällt: 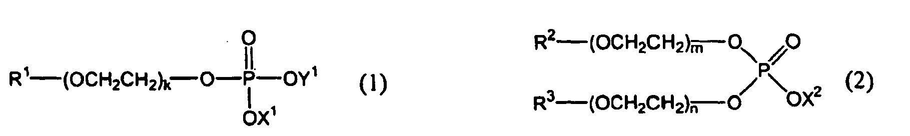 worin R¹, R² und R³ jeweils eine lineare oder verzweigte Alkyl- oder Alkenylgruppe mit 8 bis 18 Kohlenstoffatomen sind; X¹, X² und Y¹ jeweils ein Wasserstoffatom, Alkalimetallatom, Erdalkalimetallatom, Alkanolamin oder Ammonium sind und k, m und n jeweils eine Durchschnittszahl der zugegebenen Mole ist und von 0 bis 10 sind.

4. Reinigungszusammensetzung nach einem der Ansprüche 1 bis 3, weiterhin umfassend als Komponente (E) zumindest einen Glycerylether mit einer Alkyl- oder Alkenylgruppe mit 4 bis 12 Kohlenstoffatomen.

5. Reinigungszusammensetzung nach einem der Ansprüche 1 bis 4, weiterhin umfassend als Komponente (F) eine Verbindung dargestellt durch die folgende Formel (3):
R⁴O-(AO)ₚ-R⁵ (3)
worin R⁴ eine lineare oder verzweigte Alkyl- oder Alkenylgruppe mit 6 bis 10 Kohlenstoffatomen ist, R⁵ ein Wasserstoffatom oder Methylgruppe ist, AO eine Alkylenoxygruppe mit 2 bis 4 Kohlenstoffatomen ist und p eine durchschnittliche Zahl der zugegebenen Mole ist und eine Zahl von 0,5 bis 3 ist.

6. Reinigungszusammensetzung nach einem der Ansprüche 1 bis 5, worin eine 20-fache Verdünnung der Reinigungszusammensetzung einen pH von 4 bis 8 hat.

7. Reinigungszusammensetzung nach einem der Ansprüche 1 bis 6, die zusammen mit Wasser mit einer Härte von 1 bis 30°DH verwendet wird.

8. Reinigungszusammensetzung nach einem der Ansprüche 1 bis 7, worin das verwendete Wasser einen pH von 4 bis 9 hat.

9. Reinigungszusammensetzung nach Anspruch 4, worin der Gehalt der Komponente (E) von 0,3 bis 5 Gew.-% ist.

10. Reinigungszusammensetzung nach Anspruch 5, worin der Gehalt der Komponente (F) von 0,3 bis 10 Gew.-% ist.

11. Verfahren zum Reinigen eines Körpers, umfassend die Auftragung der Reinigungszusammensetzung wie in einem der Ansprüche 1 bis 10 beansprucht, auf den Körper.

12. Verwendung der Reinigungszusammensetzung wie in einem der Ansprüche 1 bis 10 beansprucht, zum Reinigen eines Körpers.

## Revendications

1. Composition détersive, comprenant les composants (A), (B), (C) et (D) suivants :
(A) des particules se délitant pour détersif obtenues par liaison et agglomération de particules primaires, au moins une partie desquelles est insoluble dans l'eau avec un liant soluble dans l'eau et ayant une taille moyenne de particules de pas moins de 30 µm, mais de moins de 100 µm,
(B) un sel soluble dans l'eau choisi parmi des sels inorganiques de chlorures, sulfates et carbonates, des sels organiques d'acide citrique, d'acide succinique, d'acide maléique, d'acide fumarique et d'acide malique, et des agents tensioactifs anioniques, dans laquelle le sel soluble dans l'eau est contenu dans une quantité de pas moins de 1,5% en poids, mais inférieure à la solubilité à saturation,
(C) un agent tensioactif à l'exception de l'éther glycérylique, et
(D) de l'eau,
dans laquelle le terme « insoluble dans l'eau » signifie que la solubilité des particules à 25°C est inférieure à 0,5 g dans 100 g d'eau,
dans laquelle le terme « soluble dans l'eau » signifie que la solubilité des particules à 25°C n'est pas inférieure à 0,5 g dans 100 g d'eau, et
dans laquelle la taille moyenne de particules est la taille médiane telle que déterminée en utilisant un analyseur de distribution de tailles de particules par diffraction/diffusion laser.

2. Composition détersive selon la revendication 1, dans laquelle les particules se délitant se délitent lorsque la concentration du sel soluble dans l'eau dans une solution aqueuse contenant le sel soluble dans l'eau diminue.

3. Composition détersive selon la revendication 1 ou 2, dans laquelle le composant (C) est un agent tensioactif phosphate qui est un mélange de (c1) un monoester de phosphate représenté par la formule (1) et (c2) un diester de phosphate représenté par la formule (2) et les Composants (c1) et (c2) sont contenus à hauteur d'un rapport en poids (c1)/(c2) tombant dans une plage de 100/0 à 50/50 : 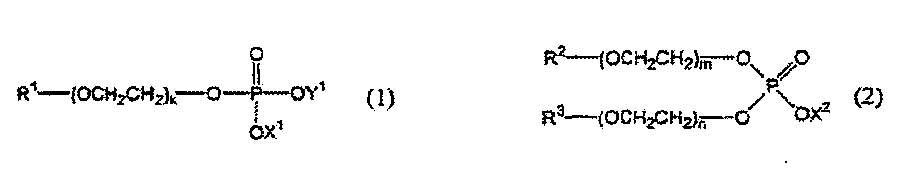 dans lesquelles R¹, R² et R³ représentent chacun un groupe alkyle ou alcényle linéaire ou ramifié ayant 8 à 18 atomes de carbone, X¹, X² et Y¹ représentent chacun un atome d'hydrogène, un atome d'un métal alcalin, un atome d'un métal alcalinoterreux, une alcanolamine ou un ammonium, et k, m et n représentent chacun un nombre moyen de moles ajoutées et valent de 0 à 10.

4. Composition détersive selon l'une quelconque des revendications 1 à 3, comprenant en outre, comme Composant (E), au moins un éther glycérylique ayant un groupe alkyle ou alcényle ayant 4 à 12 atomes de carbone.

5. Composition détersive selon l'une quelconque des revendications 1 à 4, comprenant en outre, comme Composant (F), un composé représenté par la formule (3) suivante :
R⁴O-(AO)ₚ-R⁵ (3)
dans laquelle R⁴ représente un groupe alkyle ou alcényle linéaire ou ramifié ayant 6 à 10 atomes de carbone, R⁵ représente un atome d'hydrogène ou un groupe méthyle, AO représente un groupe alkylèneoxy ayant 2 à 4 atomes de carbone et p représente un nombre moyen de moles ajoutées et est un nombre de 0,5 à 3.

6. Composition détersive selon l'une quelconque des revendications 1 à 5, dans laquelle une dilution 20 fois de la composition détersive a un pH de 4 à 8.

7. Composition détersive selon l'une quelconque des revendications 1 à 6, qui est utilisée en même temps que de l'eau ayant une dureté de 1 à 30°DH.

8. Composition détersive selon l'une quelconque des revendications 1 à 7, dans laquelle l'eau utilisée a un pH de 4 à 9.

9. Composition détersive selon la revendication 4, dans laquelle la teneur du Composant (E) est de 0,3 à 5% en poids.

10. Composition détersive selon la revendication 5, dans laquelle la teneur du Composant (F) est de 0,3 à 10% en poids.

11. Procédé de nettoyage d'un corps, qui comprend l'application de la composition détersive selon l'une quelconque des revendications 1 à 10 au corps.

12. Utilisation de la composition détersive selon l'une quelconque des revendications 1 à 10 pour nettoyer un corps.
